# EUROPEAN PATENT SPECIFICATION

(11) **EP 2 779 144 B1**
(45) Date of publication and mention of the grant of the patent: **01.11.2017**
(21) Application number: 13158858.4
(22) Date of filing: 12.03.2013
(51) Int. Cl.: G09B 23/28, A61B 5/00

(54) **Device for simulating blood flow**
Vorrichtung zur Blutflusssimulation
Dispositif pour simuler l'écoulement sanguin

(43) Date of publication of application: 17.09.2014
(73) Proprietor: Université de Mons, 7000 Mons (BE); Université Libre de Bruxelles, 1050 Bruxelles (BE)
(72) Inventor: Chodzynski, Kamil Jerzy, B-7000 MONS (BE); Zouaoui-Boudjeltia, Karim, B-6540 LOBBES (BE); Coussement, Grégory, B-7870 LENS (BE)
(74) Representative: Pronovem

(56) References cited:
- CN-A- 101 347 360
- CN-A- 101 430 311
- DE-A1- 4 035 452
- FR-A1- 2 973 146
- GB-A- 1 404 756
- GB-A- 2 315 905
- US-A1- 2011 045 451

## Description

### Field of the invention

The present invention is related to a device for *in vitro* simulating blood flow in vessels.

The present invention may be used to study biological events, and in particular, pathological diseases related to blood flow such as cardiac diseases, including atherosclerosis and spontaneous or stent-induced thrombosis in intracranial aneurysms.

The present invention may be used to study flow phenomena in different geometry of vessels such as straight pipes, bifurcations as well as different type of more complex vessel geometries (aneurysms, stenosis...).

The present invention may be used to study flow phenomena effect in aneurysms or stenosis with a mesh 'tube' inserted, called the stent.

The present invention may be used to test the effect of potential drugs or other molecules or compositions.

### Background of the invention

An aneurysm is a bubble-like extension of the wall of a blood vessel provoked usually by pathologies or mechanic disturbances of the blood flow. Aneurysms are considered dangerous because they can rupture and provoke internal haemorrhages that can lead to a stroke and even dead.

Nowadays, once an unrupted aneurysm has been detected, physicians have to rely entirely in their experience and reported cases to evaluate the chances of the aneurysm to rupture and what type of therapeutic approach to choose.

Little is known about the healing mechanism, namely the formation of a clot inside the cavity after insertion of a stent, the only widely accepted dogma by the medical community is that the hemodynamic forces exerted by the blood flow in the affected region are key factors.

Cardiovascular diseases, and in particular atherosclerosis, are known to be among the main causes of mortality and morbidity in the world, especially in developed countries.

Atherosclerosis is a chronic inflammatory disease of large and medium arteries, leading to the accumulation of cholesterol and white blood cells at the subendothelial level in the blood vessels, thereby resulting into the thickening of the wall and the narrowing of the lumen. It may be accompanied by complications, such as a myocardial infraction or thrombosis, with sudden hemostatic blockage inside a blood vessel

Most physicians agree on the fact that factors such as diabetes, stress, hypertension or hypercholesterolemia are systemic risk factors causing the development of this disease, but the factor triggering atherosclerosis still remain unknown.

However, recent *in vivo* and *in vitro* studies on atherosclerosis show that atherosclerotic lesions form at specific regions of the arterial tree, which are associated with local variations in arterial hemodynamic properties.

In other words, there are places in the vasculature where the accumulation of cholesterol and macrophage white blood cells has a higher probability of appearing. These places are namely branches and junctions which cause perturbations in the blood flow, with change of the shear stress level along endothelial cells of the vessels, which may lead to an abnormal response of endothelial cells. Moreover, it is also known that endothelial cells have the ability to change their morphology as well as their biology, including their gene expression, depending on the friction forces exerted on them.

This seems to indicate that mechanical forces driven by blood flow, and in particular by the pulsatile behaviour of coronary blood flow in the vessels, may be a key factor in the development of atherosclerosis.

*In vitro* studies on cell cultures have already been performed, but most of them bring only limited information because they fail to mimic the complexities of the *in vivo* environment.

The document FR 2 973 146 discloses a test bench having a non-volumetric centrifugal pump including an input connected to a blood tank and an output connected to a supply pipe for feeding test tubes connected to a return fluid collector. An electronic control unit controls power supply of the pump to reproduce systolic and diastolic phases of a cardiac cycle.

In document CN 101 347 360, the pulsating flow is obtained by using a proportional valve controlled by an electronic controller to produce the flow variations, thereby obtaining a liquid pulsating source.

### Aims of the invention

A device offering a realistic *in vitro* model based on numerical simulation of human blood flows in vessels would then be very useful for studying this pathology and for testing potential solutions, and in particular potentially active substances, and would be an attractive alternative to *in vivo* testing.

An aim of the invention is to reproduce particular parts of circulatory system together with the physiological conditions as precisely as possible. More particularly, an aim of the invention is to reproduce the flow conditions in a particular vessel.

### Summary of the invention

The present invention provides a device for *in vitro* simulation of blood flows. The liquid used for the simulation can be the whole blood (in that case, along with anti-clotting agent to avoid clotting in the device) or a suitable aqueous solution, preferably maintained to physiological pH, and preferably containing suitable nutrient.

The present device is based on numerical simulation, and in particular on computational fluid dynamic (CFD) calculations.

More precisely, said device comprises a closed-loop hydraulic circuit for circulating a liquid from a reservoir through a test chamber in a pulsatile manner, whereby the liquid may be continuously recirculated through the device.

More particularly, the present invention is related to a closed-loop hydraulic circuit for *in vitro* simulation of blood flow and for testing the effect of such simulated flow on a biological sample, comprising:
- a tank for storing a flowing liquid;
- a constant flow pump for pumping the liquid out of the tank for imposing, in use, an average constant flow in the hydraulic circuit;
- a pulsating pump for superimposing to said average constant flow a variable flow having a zero average flow rate;
- a test section for receiving a biological sample to be tested under flow conditions imposed by the said means;
- means for regulating the pressure downstream of the test section;
- pressure and flow sensors for sensing pressure and flow conditions in the test section;
- means for measuring and collecting biological data in the test section;
- a control unit comprising regulation means for controlling the constant flow pump and the pulsating pump.

According to particular preferred embodiments, the present invention comprises one or a suitable combination of at least two of the following features:
- the control unit comprises a closed loop regulation system, adapting in real-time the input parameters of the constant flow pump, the pulsating pump and the means for regulating the pressure for reproducing a target pulsating pressure and flow waveform;
- the means for regulating the pressure comprises a first and a second proportional pressure valve, the first proportional pressure valve controlling the average pressure, and the second proportional pressure valve controlling a superimposed pulsating pressure component;
- the constant flow pump comprises a centrifugal pump;
- the pulsating pump comprises a cylinder and a moving piston connected on a side branch of the hydraulic circuit, said cylinder preferably comprising a diaphragm such as a rubber diaphragm;
- the closed-loop hydraulic circuit comprises a one-way valve between constant flow pump and the pulsating pump for avoiding backflow to the tank;
- the tank comprises means for regulating the temperature of the liquid contained therein;
- the tank comprises means for regulating the oxygen level and pH in the liquid, the pH being preferably regulated by means for regulating Carbon dioxide concentration
- the tank comprises means for regulating supplements such as nutrients and/or salts concentration in the liquid, ;
- the test section comprises a transparent wall and the means for measuring and collecting biological data in the test section comprises a microscope to observe the sample in real time;
- the microscope is a digital holographic microscope to observe the sample in three dimensions;
- the microscope has fluorescence observation capabilities.

another aspect of the invention is related to a cyclic method for regulating the flow in the system according to any of the previous claim, each cycle comprising the steps of:
- providing a flow reference waveform;
- splitting said flow reference waveform into a reference average flow and a pulsatile reference flow waveform;
- imposing an average constant flow in the hydraulic circuit by running the constant flow pump at a predetermined rate;
- imposing a pulsating flow by running pulsating pump, using predetermined pulsating pump parameters;
- measuring the flow in the hydraulic circuit;
- calculating the average flow in the cycle;
- comparing the calculated average flow in the cycle to the reference average flow to obtain an error average flow;
- comparing the reference flow waveform to the measured flow waveform to obtain an error pulsating flow;
- adapting the predetermined pumping rate of the constant flow pump, by minimising the error average flow;
- adapting the predetermined parameters of the pulsating pump by minimising the error pulsating flow.

Preferably the adapting steps are performed using proportional and integral (PI) regulation method.

Advantageously, the pulsating pump is controlled by using an Internal Model Controller.

Advantageously, the pulsating pump comprises a cylinder and a moving piston, the cyclic method comprising the step of calculating the average position of the piston and comparing the calculated average position with the value calculated in the previous cycle, and adapting the constant flow pump rate in case of variation of said average position in order to avoid drifting of the piston position from one cycle to the next cycle.

### Definitions and abbreviations

In the present description, "endothelial cells" refer to the cells lining all blood vessels.

It is meant by "hemodynamic forces" the mechanical forces due to blood flow.

It is meant by "shear stress" the tangential frictional force acting in the direction of flow along a vessel's surface.

It is meant by "wall shear stress" the fluid frictional force per unit of surface area. Wall shear stress acts along the blood vessel's longitudinal axis.

### Brief description of the Figures

Figure 1 represents a schematic view of a hydraulic circuit and sensors according to the invention.
Figure 2 represents a schematic view of the regulation loops of an example of the invention.

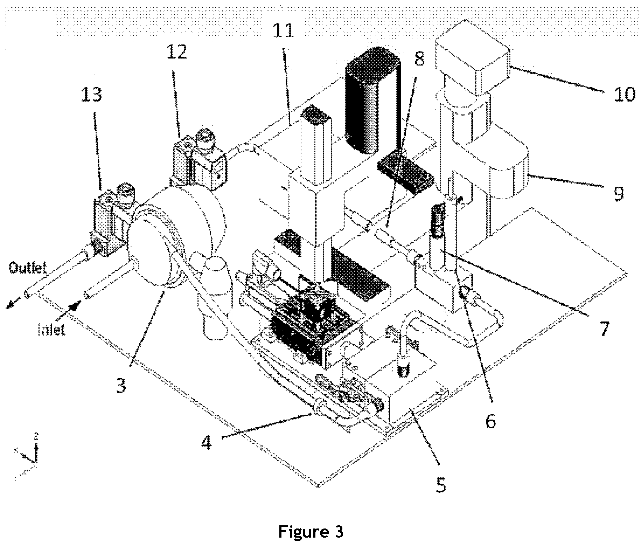

Figure 3 represents a portable version of the closed-loop hydraulic circuit of the invention. This version is intended to be enclosed in a transparent box.
Figure 4 represents the electrical parts controlling the hydraulic circuit.

### Figure keys

1 - reservoir
2 - incubator
3 - centrifugal pump
4 - one-way valve
5 - piston pump
6 - thermocouple
7 - pressure sensor
8 - test section
9 - microscope
10 - camera
11 - flowmeter
12, 13 - solenoid proportional valves
14 - AC/DC controller
15 - heating system
16 - thermocouple
17 - Control unit (PC class computer / notebook / touch screen computer).

### Detailed description of the invention

The device according to the present invention offers different technical advantages.

The present device is useful in helping to determine how fluid dynamics modulates the response of endothelial cells under physiological conditions in an *in vitro* flow loop.

The present device permits critical examination of the effects of critical flow parameters on endothelial cells on the surfaces of vessels.

In particular, the present device simulates shear stress, the tangential frictional force acting in the direction of flow along a vessel's surface.

The present device is adapted to reproduce different kinds of vessels, different either by their dimensions and/or by their geometry, with minor adaptations.

This device is also adapted to different kinds of support measurement equipments including, but not limited to lasers such as for example Participle Image Velocimetry (PIV) lasers, scanners such as for example Computer Tomography (CT) scanners, microscopes such as Digital Holographic Microscopes.

Moreover, this device is easy to use, compact, mobile, as automatic as possible.

At least the hydraulic part of this device may easily be sterilized, without any damage to its functioning, even after several sterilization cycles.

According to the present invention, the flow is splitted in a continuous component and a variable component having an average zero value. The splitted flow is then imposed by using a constant flow pump having an essentially constant flow rate (i.e. the variation is slow in comparison with the normal cycle time) and a pulsating pump having an average zero flow rate. Such combination of pumps is particularly suitable to reproduce a pulsating flow of liquid inside the closed circuit, such that a fluid-dynamic component is introduced into the system which makes the flow in the test chamber very similar to the conditions present in the coronary flows, reproducing in a reliable manner what happens in the vessel circuit in the vicinity of heart.

More particularly, such splitting of the flow in an average component and a superimposed pulsating component makes the regulation easier by using separated regulation loops.

To reproduce the mean flow rate, either a peristaltic pump or a centrifugal pump can be used.

The peristaltic pump can drive a nearly constant mean flow rate.. But by its working principle, based on the compression of a pipe by rotating round pebble, that peristaltic pump can destroy cells, like Red Blood Cells, by the rolling process of the pipe. Moreover, as it presses pipes, little pieces of them can be released to the system and make the medium toxic for the living cells. The peristaltic pump is a roller positive displacement pump that gives a flow rate oscillating around the mean flow rate. In order to reach a more constant flow rate it is necessary to add a second pump head or/and a compliance chamber. However, the compliance chamber incorporates more elasticity to the system and has a bad impact on the control capabilities.

The centrifugal pump avoids that rolling inconvenient and can be used to provide the mean flow rate without destroying the cells if necessary for the use of real Red Blood Cells in the medium. However the centrifugal pump is more influenced by movements of the piston pump: it has an impact of the pumping flow behaviour especially on the pick flow.

Pumping means are adapted for altering and changing hemodynamic conditions (pressure and flow variables) in the loop so as to permit simulating the hemodynamic conditions to capillaries of the human circulatory system.

The device comprises different controlling means: means for measuring, monitoring and controlling different flow conditions by creating, measuring, monitoring and controlling flow variables (paramaters) such as the fluid temperature, the fluid pressure, the fluid pulse characteristics (including pulse frequency, pulse width and more generally pulse waveform) and the flow rate through the device.

The liquid or fluid used in the present invention preferably comprises nutrients necessary to support metabolism of the living tissues present in the test chamber.

The temperature of the liquid or fluid should preferably be maintained at about 37° C, and its pH at about 7.45.

Preferably, although nutrient replacement should not be required over short-term experiments (1-2 days), gas exchange will be necessary to maintain a good oxygenation and regulate carbon dioxide levels.

In order to avoid introducing potentially harmful impurities into the system, all materials that may contact the liquid or fluid may be sterilized according to conventional techniques.

Care should be paid that the different elements of the device will not damage living cells when the device is operating. Appropriate controlling means should be included into the device for that purpose.

Before any testing assay, the overall performance of the device, and in particular of the loop, should be preliminary tested to verify that the desired flow conditions can be achieved.

The fact that the circuit (the loop) is closed further offers the advantage to prevent any liquid leakage from the device, thereby reducing the risk of contamination to the operators, and increasing the overall conditions of cleanliness and hygiene.

According to the present invention, the response of endothelial cells are preferably monitored in the test chamber by acquisition of data, for example images, by optical acquisition means, which advantageously comprises at least a microscope, optionally connected to filming means such as for example a camera, and in particular a CCD camera.

More advantageously, the filming means comprises an integrated recording/acquisition system connected to electronic processing means, such as for example a digital computerized system with a memorized program.

Preferably, said memorized program is such that it is able, when executed in the computerized system, to analyze in real time biological parameters such as the morphological, kinetic, and biochemical variations inside and outside the endothelial cells in test chamber in response to different conditions of mechanical forces.

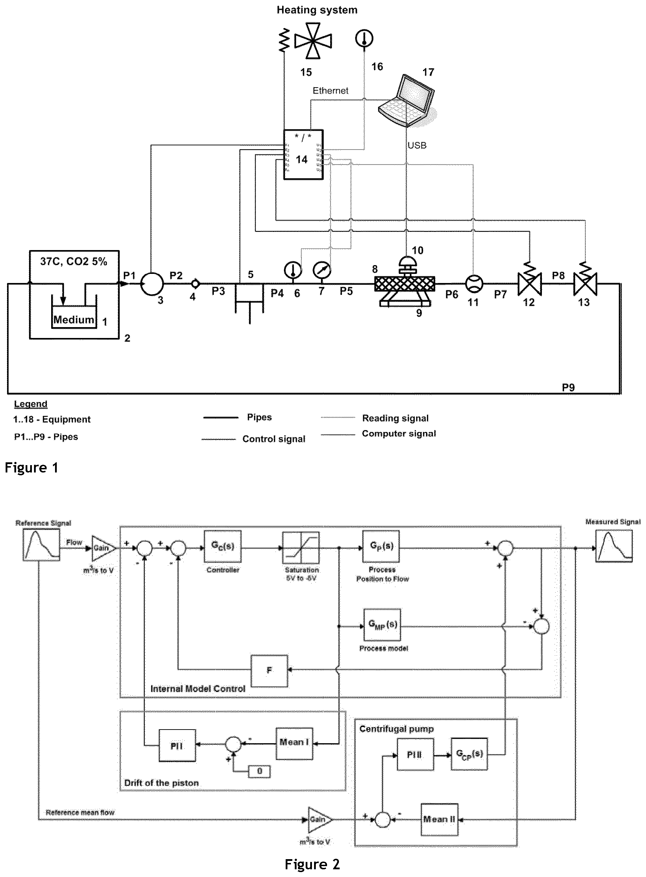

The principle of the control loop is shown in Fig 2. The reference signal is divided into two signals: the mean flow and the pulsatile fluctuations of the flow. From that division, we have two main controls. In the first control (Centrifugal pump in the Fig.2), the mean flow is controlled by the PI controller number II and the function "mean II".

The function "mean II" is responsible for calculating the mean flow at each cycle. It adds every point of the measured flow cycle and divides the latter by the number of the measured samples for this cycle. Then the signal is sent as a measured signal to the adder in order to compare with the mean reference value. Then the controller PI II set rotation of the centrifugal pump in order to get the desired mean flow rate (the transfer function G_{CP}(s)). Thanks to the function "mean II" the control loop is less sensitive to disturbances on the mean flow, which helps to achieve the stability of the whole system.

The second control loop is responsible for controlling the shape of the pulsatile flow. This loop is based on the Internal Model Control (Internal Model Control in the Fig.2) and is additional to the PI controller.

Basically, the reference signal is compared with a measured signal and is sent to the internal controller Gc(s). After that, the controlled signal goes through a saturation block which sets the movement limits of the linear motor. The signal is then sent to the controller of the linear motor and drives the movements. Later the flow is read by the electromagnetic flowmeter that is presented in the Fig. 2 as a transfer function between position of the linear motor and flowmeter G_{P}(s) and in addition read by model process G_{MP}(s) in order to predict position of the linear motor.

After that the signal is preferably treated by a filter F (used only in real-time process) and then compared with the actual position of the piston. An additional filter is used because the noise of the read signal from electromagnetic flowmeter can be too high and the mean flow rate pumped by the centrifugal pump and read by the flowmeter is not perfectly constant and this may have influence on the controlled signal.

In addition, in order to prevent the linear motor from drifting, an additional loop (Drift of the piston in the Fig.2) is advantageously incorporated into the regulation system. This loop calculates a mean position for every cycle separately and compares it with the mean position previously set. If there is any drift, the PI controller number II modifies the reference signal of the flow.

To determine adjustments/parameters of the PI controllers the Ziegler-Nichols method was used.

To be able to control the whole system a transfer function of the device of the invention has to be found. One of the methods is the characteristic of the frequency response of the system. This characteristic shows how amplitude and phase change after passing through the whole systems. The typical input signal x(t) used as the excitation signal is a sinusoidal wave (known also as a sweep signal or a chirp signal) with amplitude A and frequency ω. Then, the output signal y(t) coming from the system is also a sinusoidal wave but with different amplitude B, the same frequency ω and a phase shift ϕ between the input signals.

The input signal is a position of the linear motor in volts which drives a piston pump with a diaphragm. The input signal, a sinusoidal wave with amplitude A=0.5V and frequency ranging between 0.1Hz to 25 Hz with a step frequency 0.1Hz was put. The output signal is the flow read in volts. Based on the experimental data the magnitude and phase are then calculated.

To determine the unknown parameters in the transfer function that is based on the experimental data, two methods may be used: one is to determine the zeros and poles of the system by observation of the experimental data; second is based on the known transfer function for which parameters can be found by used the parameter estimation function that can be used from the commercial software such as Simulink® Design Optimization™ toolbox. Five orders transfer function with delay was are preferably used to get a sufficient fitting.

The calculated transfer functions are then used to determine the parameters of an internal model controller used for controlling the pulsating component of the pulsating pump.

### Example of one embodiment

A preferred embodiment of the device according to the present invention is presented on Figure 1. The closed-loop hydraulic circuit starts in an incubator 2 where a reservoir 1 with bio-medium is put in order to maintain the physiological condition (pH level at 7.45). This is done by keeping gas balance at the proper level i.e. CO2 5% in the air.

Then the mean flow rate is pumped by a centrifugal pump 3 which is particularly designed to pump blood, for example BPX-80 Biopump (Medrotronic).

Downstream of that pump, there is a one-way valve 4 and a piston pump 5 connected to a linear motor that controls oscillations over the mean flow rate.

The temperature level 37°C is read on the thermocouple 6. Just upstream of a test section 8, there is a pressure sensor 7 which measures the pressure inside the system.

The test section 8 is on-line with a microscope 9 which is connected to a digital camera 10. The camera takes pictures of ECs while the system is working. After that, the flow rate is measured by a flowmeter 11. The last two pieces of equipment are solenoid proportional valves 12, 13 for controlling the pressure in the circuit.

For obtaining sterility conditions as well as for the mobility of the system, all equipment is enclosed in a plastic transparent box.

The temperature of the medium is controlled by incorporating a heater in the system 15 and an additional thermocouple 16 inside the box.

The whole system is controlled by National Instruments™ real-time CompactRIO controller under LabVIEW™19 environment. The preliminary control strategy is based on the closed loop with Internal Model Control and supported PI controllers.

The closed loop regulation strategy opposite the open loop, can give feedback to the changes of the environment such as temperature, viscosity, flow or pressure perturbation and others. The LabVIEW™ designed software is a great tool to build a friendly graphical interface that will be easy to understand and control physiological flow conditions by future investigators.

Examples of technical characteristics of these hydraulic elements are presented in the Table hereafter:

| **Hydraulic element** | **Technical characteristics** |
|---|---|
| Centrifugal pump | External drive motor model 540T with the pump head BP-50, Medtronic; |
| Check valve | W96029 x10 Clapet Kynar 6,4x6,4mm |
| | check valve |
| Piston pump | a piston chamber made of Plexiglas with a diameter of 24 [mm], a diaphragm (D-94-81, Diacom) and a linear motor (High-Performance Stator PS01-23x80F-HP-R and High Performance slider for H-Guide PL01-12x200/160-HP, Linmot) |
| Temperature sensor | RTD thermocouple (PR-11-2-100-M60-100-E-CLA, Omega) |
| Pressure sensor | RVAQ300GU2, Sensortechnics |
| Test compartment or test chamber | Plexiglas pipe with an internal diameter of 4 [mm] and a length of 60 [mm], which gives an internal surface of 7.54 cm² |
| Microscope | DMIL, Leica |
| Digital camera | Infinity 2-5C, Lumenera |
| Electromagnetic flowmeter | MM-04-XX-VD-SS-XX-0000-22-1A-1F-00-2R-00-SK, Minimag |
| Solenoid valves | Proportional solenoid valves type 2833, with a digital control electronic for proportional valves, type 8605, Bürkert Contromatic SA |
| Pipes | Type of pipes is a Tygon B-44-4X with following dimentions: SN-95643-13,ID-6.4 mm and SN-95643-09,ID-4.8 mm, Masterflex |
| Fittings | SN-31304-10 Quick-disconnect fittings, SN-06360-82 Quick-Disconnect Hose Barb Insert, SN-06360-47 Quick-Disconnect |
| | Hose Barb Insert, SN-06360-44 Quick-Disconnect Hose Barb Insert, SN-06361-00 Quick-Disconnect NPT(M) Pipe Adapter, SN-06361-01 Quick-Disconnect NPT(M) Pipe Adapter, SN-06361-42 Quick-Disconnect NPT(M) Pipe Adapter, SN-34006-17 Straight Unions, 1/4, John Guest acetal push-to-connect straight unions, Masterflex |

Different types of endothelial cells may be placed in the test compartment. In this example, endothelial cells of type EA.hy926 were used.

Alternatively, a multisheet culture system containing both endothelial cells and other cells such as vascular smooth muscle cells may also be placed in test compartment.

The test compartment is advantageously transparent so as to allow observation under the microscope.

The test compartment may also be adapted to recording of flow parameters and cell harvest after treatments.

The hydraulic unit further comprises a tank or incubator (Revco Ultima II RMI3000 Series Midi CO₂ incubator with automatic CO₂ control, Thermo Scientific). The incubator functions so as to keep the conditions at 37°C and 5% of CO₂.

A tank in the incubator is able to be filled with a fluid or liquid able to mimic blood and serve as a cell culture medium. For example, as fluid a medium DMEM with 1g Glucose, 25mM Hepes and supplemented with: 10% FBS (Foetal Bovine Serum), 1% L-Glutamine, 1% Penicillin + Streptomycin, 2% HAT (Hypoxanthine Aminoptein Thymidine) and 1% NEAA (Non essential amino acid) may be used. In order to set viscosity at a desired level, a Dextran complex may also be added to this medium.

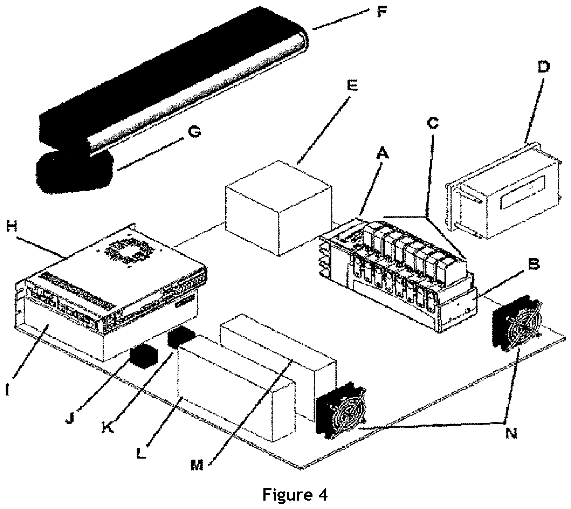

The electrical equipment is represented in figure 4 and comprises:
- a real-time controller (A),
- a chassis with FPGA (B),
- modules of the real-time system (C),
- a controller of the electromagnetic flowmeter (D),
- a centrifugal pump controller (E),
- a UV lamp (F) as sterilisation means,
- a heater 12V and 15A (G),
- a linear motor controller (H) as means for driving the linear motor, controlling the position of the piston,
- a linear motor power supply (I),
- a relay 24V and 20A (J, K),
- a 12V power supply (L),
- a 24V power supply (M),
- 24V fans (N) as cooling means for cooling the electrical unit

The technical characteristics of these electrical elements are presented in the Table hereafter:

| **electrical element** | **Technical characteristics** |
|---|---|
| Real-time controller | NI9024, National Instrument™ |
| | 800 MHz processor, 4 GB nonvolatile storage, 512 MB DDR2 memory. On the controller runs LabVIEW™ Real-Time for deterministic control, data logging, and analysis. |
| chassis with FPGA | reconfigurable chassis, N19112, National Instrument™ |
| modules | NI9265, NI9203, NI9215, 2x NI9217, NI9263, NI9472, NI9481, National |
| | Instrument™, |
| | with read/write analogue/digital inputs such as (+/- 10V, RTD thermocouple and 0-20mA) |
| Centrifugal pump controller (controls the rotations of the centrifugal pump) | palatine controller external drive motor 90446/99/02, Medtronic |
| linear motor controller | E1250-PL-UC, Linmot |
| a linear motor power supply | NT01-72/240, Jung Antriebstechnik u. automation GMBH |
| 12V power supply | PSIN30012 |
| 24V power supply | PSIN30024 |
| UV lamp | UV germicidal lamp; 15 watts, wavelength 254 nm, 31µW/cm² intensity, Masteflex |
| heater | 150W, 12V, 15A |
| fans | size 80x80x25mm, 24V |

The heating system or heater is able to keep the temperature of the medium in desired physiological level of about 37°C. In order to monitor the temperature inside of the box, the device is advantageously provided with a four air thermocouple such as a RTD-2-F3105-120-T thermocouple, Omega (not shown).

All the equipment, i.e. both the hydraulic unit and the electrical unit, is/are advantageously controlled by processing means including a PC computer, preferably with Labview™ Core Software and Windows XP operation system. For example, as a PC computer a Dell™ Inspiron 9400 with 4 GB ram and Intel Core 2 Duo T5800 1.83 GHz may be used.

Based on CFD simulations, the processing means are able to control the signals generated by the operating unit that will be applied to the cells in the test chamber, and that will mimic *in vitro* as closely as possible the signals encountered in the blood vessels *in vivo.*

## Claims

1. Closed-loop hydraulic circuit for *in vitro* simulation of blood flow and for testing the effect of such simulated flow on a biological sample, comprising:
- a tank (1) for storing a flowing liquid;
- a constant flow pump for pumping the liquid out of the tank for imposing, in use, an average constant flow in the hydraulic circuit;
- a test section (8) for receiving a biological sample to be tested under flow conditions;
- Means for regulating the pressure downstream of the test section;
- Pressure and flow sensors (7,11) for sensing pressure and flow conditions in the test section;
- Means for measuring and collecting biological data in the test section;
**characterised in that** the closed loop circuit further comprises
- a pulsating pump for superimposing to said average constant flow a variable flow having a zero average flow rate;
- a control unit (17) comprising regulation means for controlling the constant flow pump and the pulsating pump.

2. Closed-loop hydraulic circuit according to claim 1 wherein the control unit (17) comprises a closed loop regulation system, adapting in real-time the input parameters of the constant flow pump, the pulsating pump and the means for regulating the pressure for reproducing a target pulsating pressure and flow waveform.

3. Closed-loop hydraulic circuit according to any of the previous claims wherein, the means for regulating the pressure comprises a first and a second proportional pressure valve (12,13), the first proportional pressure valve (12) controlling the average pressure, and the second proportional pressure valve (13) controlling a superimposed pulsating pressure component.

4. Closed-loop hydraulic circuit according to any of the previous claims wherein the constant flow pump comprises a centrifugal pump (3).

5. Closed-loop hydraulic circuit according to any of the previous claims wherein the pulsating pump comprises a cylinder and a moving piston (5) connected on a side branch of the hydraulic circuit.

6. Closed-loop hydraulic circuit according to any of the previous claims comprising a one-way valve (4) between constant flow pump and the pulsating pump for avoiding backflow to the tank (1).

7. Closed-loop hydraulic circuit according to any of the previous claims wherein the tank (1) comprises means for regulating the temperature of the liquid contained therein.

8. Closed-loop hydraulic circuit according to any of the previous claims wherein the tank comprises means for regulating the oxygen level and pH in the liquid, the pH being preferably regulated by means for regulating Carbon dioxide concentration.

9. Closed-loop hydraulic circuit according to any of the previous claims wherein the test section comprises a transparent wall and the means for measuring and collecting biological data in the test section comprises a microscope (9) to observe the sample in real time.

10. Closed-loop hydraulic circuit according to any of the previous claims the microscope (9) is a digital holographic microscope to observe the sample in three dimensions.

11. Closed-loop hydraulic circuit according to claim 9 or 10 wherein the microscope (9) has fluorescence observation capabilities.

12. Cyclic method for regulating the flow in the system according to any of the previous claim, each cycle comprising the steps of:
- providing a flow reference waveform;
- splitting said flow reference waveform into a reference average flow and a pulsatile reference flow waveform;
- imposing an average constant flow in the hydraulic circuit by running the constant flow pump at a predetermined rate;
- imposing a pulsating flow by running pulsating pump, using predetermined pulsating pump parameters;
- measuring the flow in the hydraulic circuit;
- calculating the average flow in the cycle;
- comparing the calculated average flow in the cycle to the reference average flow to obtain an error average flow;
- comparing the reference flow waveform to the measured flow waveform to obtain an error pulsating flow;
- adapting the predetermined pumping rate of the constant flow pump, by minimising the error average flow;
- adapting the predetermined parameters of the pulsating pump by minimising the error pulsating flow.

13. Cyclic method according to claim 12 wherein the adapting steps are performed using proportional and integral (PI) regulation method.

14. Cyclic method according to any of claims 12 or 13 wherein the pulsating pump comprises a cylinder and a moving piston, the cyclic method comprising the step of calculating the average position of the piston and comparing the calculated average position with the value calculated in the previous cycle, and adapting the constant flow pump rate in case of variation of said average position in order to avoid drifting of the piston position from one cycle to the next cycle.

15. Cyclic method according to any of claims 12 to 14 wherein the pulsating pump is controlled by using an Internal Model Controller.

## Patentansprüche

1. Geschlossener Hydraulikkreislauf zur in vitro-Simulation des Blutflusses und zur Untersuchung der Wirkung des simulierten Flusses auf eine biologische Probe, umfassend:
- einen Behälter (1) zum Einschließen einer fließenden Flüssigkeit;
- eine Konstantpumpe zum Pumpen der Flüssigkeit aus dem Behälter, um im Einsatz einen durchschnittlich stetigen Fluss im Hydraulikkreislauf zu erzwingen;
- einen Untersuchungsabschnitt (8) zur Aufnahme einer unter Flussbedingungen zu untersuchenden biologischen Probe;
- Mittel zum Regeln des Drucks stromabwärts vom Untersuchungsabschnitt;
- Druck- und Durchflusssensoren (7, 11) zur Erfassung von Druck- und Flussbedingungen im Untersuchungsabschnitt;
- Mittel zum Messen und Erfassen biologischer Daten im Untersuchungsabschnitt;
**dadurch gekennzeichnet, dass** der geschlossene Kreislauf ferner umfasst:
- eine pulsierende Pumpe, um dem durchschnittlich stetigen Durchfluss einen variablen Durchfluss, dessen durchschnittlicher Durchfluss gleich Null ist, zu überlagern;
- eine Steuerung (17) umfassend Regelungsmittel zum Regeln der Konstantpumpe und der pulsierenden Pumpe.

2. Geschlossener Hydraulikkreislauf nach Anspruch 1, wobei die Steuerung (17) ein geschlossenes Regelungssystem umfasst und die Eingabeparameter der Konstantpumpe, der pulsierenden Pumpe und der Druckregelungsmittel in Echtzeit anpasst, um einen pulsierenden Druck-Sollwert und eine Zielflusswellenform zu reproduzieren.

3. Geschlossener Hydraulikkreislauf nach einem der vorstehenden Ansprüche, wobei die Druckregelungsmittel ein erstes und ein zweites Druckproportionalventil (12, 13) umfassen, wobei das erste Druckproportionalventil (12) den durchschnittlichen Druck regelt und das zweite Druckproportionalventil (13) eine überlagerte pulsierende Druckkomponente regelt.

4. Geschlossener Hydraulikkreislauf nach einem der vorstehenden Ansprüche, wobei die Konstantpumpe eine Kreiselpumpe (3) umfasst.

5. Geschlossener Hydraulikkreislauf nach einem der vorstehenden Ansprüche, wobei die pulsierende Pumpe einen Zylinder und einen beweglichen Kolben (5) umfasst, die an einen Seitenstutzen des Hydraulikkreislaufs angeschlossen sind.

6. Geschlossener Hydraulikkreislauf nach einem der vorstehenden Ansprüche, umfassend ein Rückschlagventil (4) zwischen der Konstantpumpe und der pulsierenden Pumpe, um einen Rückfluss in den Behälter (1) zu vermeiden.

7. Geschlossener Hydraulikkreislauf nach einem der vorstehenden Ansprüche, wobei der Behälter (1) Mittel zum Regeln der Temperatur der darin enthaltenen Flüssigkeit umfasst.

8. Geschlossener Hydraulikkreislauf nach einem der vorstehenden Ansprüche, wobei der Behälter Mittel zum Regeln des Sauerstoffspiegels und pH-Werts in der Flüssigkeit umfasst, wobei der pH-Wert vorzugsweise durch Mittel zum Regeln der Kohlendioxidkonzentration geregelt wird.

9. Geschlossener Hydraulikkreislauf nach einem der vorstehenden Ansprüche, wobei der Untersuchungsabschnitt eine durchsichtige Wand umfasst und die Mittel zum Messen und Erfassen biologischer Daten im Untersuchungsabschnitt ein Mikroskop (9) umfasst, um die Probe in Echtzeit zu beobachten.

10. Geschlossener Hydraulikkreislauf nach einem der vorstehenden Ansprüche, wobei das Mikroskop (9) ein holographisches Digitalmikroskop zur Beobachtung der Probe in drei Dimensionen ist.

11. Geschlossener Hydraulikkreislauf nach Anspruch 9 oder 10, wobei das Mikroskop (9) Fluoreszenzbeobachtungsfähigkeiten aufweist.

12. Zyklisches Verfahren zum Regeln des Durchflusses im System nach einem der vorstehenden Ansprüche, wobei jeder Zyklus die nachfolgenden Schritte umfasst:
- Vorsehen einer Referenz-Flusswellenform;
- Aufteilen der Referenz-Flusswellenform in einen durchschnittlichen Referenzdurchfluss und eine pulsatile Referenz-Flusswellenform;
- Erzwingen eines durchschnittlich konstanten Durchflusses im Hydraulikkreislauf, indem die Konstantpumpe bei einer vorgegebenen Rate betrieben wird;
- Erzwingen eines pulsierenden Durchflusses durch Betreiben der pulsierenden Pumpe unter Einhaltung vorgegebener Parameter;
- Messen des Durchflusses im Hydraulikkreislauf;
- Berechnen des durchschnittlichen Durchflusses im Zyklus;
- Vergleichen des berechneten durchschnittlichen Durchflusses im Zyklus mit dem durchschnittlichen Referenzdurchflusses, um einen durchschnittlichen Durchflussfehler zu berechnen;
- Vergleichen der Referenzflusswellenform mit der gemessenen Flusswellenform, um einen pulsierenden Durchflussfehler zu berechnen;
- Anpassen der vorgegebenen Pumpenleistung der Konstantpumpe durch Minimieren des durchschnittlichen Durchflussfehlers;
- Anpassen der vorgegebenen Parameter der pulsierenden Pumpe durch Minimieren des durchschnittlichen Durchflussfehlers.

13. Zyklisches Verfahren nach Anspruch 12, wobei die Anpassungsschritte nach dem proportional-integralen (PI) Regelungsverfahren ausgeführt werden.

14. Zyklisches Verfahren nach einem der Ansprüche 12 oder 13, wobei die pulsierende Pumpe einen Zylinder und einen beweglichen Kolben umfasst, wobei das zyklische Verfahren den Schritt des Berechnens der durchschnittlichen Position des Kolbens und des Vergleichens der berechneten durchschnittlichen Position mit dem im vorigen Zyklus berechneten Wert und Anpassen der Pumpenleistung der Konstantpumpe bei Abweichungen der durchschnittlichen Position, um ein Abdriften der Position des Kolbens von einem Zyklus zum anderen zu vermeiden.

15. Zyklisches Verfahren nach einem der Ansprüche 12 - 14, wobei die pulsierende Pumpe mit einem internen modellbasierten Regler gesteuert wird.

## Revendications

1. Circuit hydraulique en boucle fermée destiné à la simulation in vitro d'un flux sanguin et à tester l'effet de ce flux simulé sur un échantillon biologique, comprenant :
- un réservoir (1) destiné à stocker un liquide en écoulement ;
- une pompe à débit constant destinée à pomper le liquide hors du réservoir pour imposer, en pratique, un débit constant moyen dans le circuit hydraulique ;
- une section de test (8) destinée à recevoir un échantillon biologique à tester dans des conditions d'écoulement ;
- des moyens destinés à réguler la pression en aval de la section de test ;
- des capteurs de pression et de débit (7, 11) destinés à détecter la pression et les conditions d'écoulement dans la section de test ;
- des moyens destinés à mesurer et à collecter des données biologiques dans la section de test ;
**caractérisé en ce que** le circuit en boucle fermée comprend en outre
- une pompe pulsée destinée à superposer sur ledit débit constant moyen un débit variable présentant un débit moyen nul ;
- une unité de commande (17) comprenant des moyens de régulation destinés à contrôler la pompe à débit constant et la pompe pulsée.

2. Circuit hydraulique en boucle fermée selon la revendication 1, dans lequel l'unité de commande (17) comprend un système de régulation en boucle fermée, adaptant en temps réel les paramètres d'entrée de la pompe à débit constant, de la pompe pulsée et des moyens de régulation de la pression afin de reproduire une pression de pulsation cible et une forme d'onde d'écoulement.

3. Circuit hydraulique en boucle fermée selon l'une quelconque des revendications précédentes, dans lequel les moyens destinés à réguler la pression comprennent une première et une seconde vannes proportionnelles de pression (12, 13), la première vanne proportionnelle de pression (12) contrôlant la pression moyenne, et la seconde vanne proportionnelle de pression (13) contrôlant une composante de pression pulsée superposée.

4. Circuit hydraulique en boucle fermée selon l'une quelconque des revendications précédentes, dans lequel la pompe à débit constant comprend une pompe centrifuge (3).

5. Circuit hydraulique en boucle fermée selon l'une quelconque des revendications précédentes, dans lequel la pompe pulsée comprend un cylindre et un piston mobile (5) reliés sur une branche latérale du circuit hydraulique.

6. Circuit hydraulique en boucle fermée selon l'une quelconque des revendications précédentes, comprenant une soupape unidirectionnelle (4) entre la pompe à débit constant et la pompe pulsée afin d'éviter le reflux vers le réservoir (1).

7. Circuit hydraulique en boucle fermée selon l'une quelconque des revendications précédentes, dans lequel le réservoir (1) comprend des moyens destinés à réguler la température du liquide contenu à l'intérieur.

8. Circuit hydraulique en boucle fermée selon l'une quelconque des revendications précédentes, dans lequel le réservoir comprend des moyens destinés à réguler le niveau d'oxygène et le pH dans le liquide, le pH étant de préférence régulé par des moyens de régulation de la concentration en dioxyde de carbone.

9. Circuit hydraulique en boucle fermée selon l'une quelconque des revendications précédentes, dans lequel la section de test comprend une paroi transparente et des moyens de mesure et de collecte des données biologiques dans la section de test comprend un microscope (9) afin d'observer l'échantillon en temps réel.

10. Circuit hydraulique en boucle fermée selon l'une quelconque des revendications précédentes, dans lequel le microscope (9) est un microscope holographique numérique qui permet d'observer l'échantillon en trois dimensions.

11. Circuit hydraulique en boucle fermée selon la revendication 9 ou 10, dans lequel le microscope (9) possède des capacités d'observation par fluorescence.

12. Procédé cyclique destiné à réguler le débit dans le système selon l'une quelconque des revendications précédentes, chaque cycle comprenant les étapes consistant à :
- prévoir une forme d'onde de référence de débit ;
- diviser ladite forme d'onde de référence de débit en un débit moyen de référence et une forme d'onde de débit de référence pulsatile ;
- imposer un débit moyen constant dans le circuit hydraulique en faisant fonctionner la pompe à débit constant à une vitesse prédéterminée ;
- imposer un débit pulsé en faisant fonctionner la pompe pulsée, à l'aide de paramètres de pompe pulsée prédéterminés ;
- mesurer le débit dans le circuit hydraulique ;
- calculer le débit moyen pendant le cycle ;
- comparer le débit moyen calculé pendant le cycle avec le débit moyen de référence afin d'obtenir un débit moyen d'erreur ;
- comparer la forme d'onde de débit de référence avec la forme d'onde de débit mesuré afin d'obtenir un débit pulsé d'erreur ;
- adapter la vitesse de pompage prédéterminée de la pompe à débit constant, en minimisant le débit moyen d'erreur ;
- adapter les paramètres prédéterminés de la pompe pulsée en minimisant le débit pulsé d'erreur.

13. Procédé cyclique selon la revendication 12, dans lequel les étapes d'adaptation sont exécutées à l'aide d'un procédé de régulation proportionnelle et intégrale (P1).

14. Procédé cyclique selon l'une quelconque des revendications 12 ou 13, dans lequel la pompe pulsée comprend un cylindre et un piston mobile, le procédé cyclique comprenant l'étape de calcul de la position moyenne du piston et de comparaison de la position moyenne calculée avec la valeur calculée lors du cycle précédent, et d'adaptation du débit de la pompe à débit constant en cas de variation de ladite position moyenne afin d'éviter tout glissement de la position du piston d'un cycle au cycle suivant.

15. Procédé cyclique selon l'une quelconque des revendications 12 à 14, dans lequel la pompe pulsée est contrôlée à l'aide d'un contrôleur de modèle interne.
